# EUROPEAN PATENT SPECIFICATION

(11) **EP 3 096 917 B1**
(45) Date of publication and mention of the grant of the patent: **26.02.2020**
(21) Application number: 14815138.4
(22) Date of filing: 19.11.2014
(51) Int. Cl.: B23K 9/16, G01N 33/00, B23K 37/00, B23K 101/06, B23K 103/14, B23K 9/32, G01N 27/411, B23K 9/028, B23K 9/095, B23K 31/12

(54) **DEVICE FOR MEASURING AND COLLECTING OXYGEN CONCENTRATION DATA IN WELDING PROCESSES**
VORRICHTUNG ZUR MESSUNG UND SAMMLUNG VON SAUERSTOFFKONZENTRATIONSDATEN IN SCHWEISSVERFAHREN
DISPOSITIF DE MESURE ET DE COLLECTE DE DONNÉES CONCERNANT LA CONCENTRATION EN OXYGÈNE DANS DES PROCÉDÉS DE SOUDAGE

(30) Priority: 21.01.2014 DE 202014100241 U
(43) Date of publication of application: 30.11.2016
(73) Proprietor: Illinois Tool Works Inc., Glenview, IL 60025 (US)
(72) Inventor: TAMM, Markus, 88662 Ueberlingen (DE); FOH, Marcel, 88677 Markdorf (DE)
(74) Representative: Trinks, Ole
(86) International application number: PCT/US2014/066276
(87) International publication number: WO 2015/112242

(56) References cited:
- CN-U- 203 324 188
- DE-B3-102009 022 846
- JP-A- H07 116 836
- JP-A- H08 172 263
- JP-A- 2013 247 181
- US-A- 5 030 420
- US-A1- 2009 302 007
- US-A1- 2010 011 837
- US-B1- 6 783 054

## Description

The present invention relates to a device for measuring and collecting oxygen concentration data in welding processes, in particular in shielded arc welding, according to the preamble of independent claim 1.

Document DE102009022846 discloses optimized inert gas admission and monitoring inert gas composition and cooling temperatures of materials. The method is used in welding of zirconium and includes measuring residual oxygen content.

Document US9272353 discloses a pipeline section centering and locking system prior to welding. The system comprises an inert gas flow and pressure regulator, the function of which is based on oxygen content.

Accordingly, the present invention relates to a device for measuring and collecting oxygen concentration data in welding processes, in particular in shielded arc welding, at least one sensor element being provided for sensing the oxygen concentration of a shielding atmosphere.

Particularly in the chemical industry, high-quality welded connections are required for plants and apparatus. Used for example in this respect for the construction of reaction towers, pumps and pipes are materials such as titanium, tantalum or zirconium, which in processing require special welding conditions in order to ensure metallurgically satisfactory weld seams. It is not unusual in this case that the shielding atmosphere produced by the shielding gas must not have an oxygen content of any more than 0.001 to 0.01% (10 to 100 ppm).

In order to ensure that there is actually such a low residual oxygen content in the shielding atmosphere, it must be measured exactly. The calculation formulae that are often used in practice, based on time, amount of gas and volume, only provide approximate reference values here, which are not sufficient in many applications. Accordingly, it is desirable to provide in addition to the welding apparatus devices for measuring the oxygen content.

An essential prerequisite for consistently high seam quality is welding under controllable conditions. In this respect, it is known for example in the case of shielded arc welding to make a shielding gas flow around the weld seam during the welding operation in order to displace oxygen. The shielding gas is a chemically inert gas, which protects the weld seam from oxidation and scaling. Apart from a visual impairment, even slight oxidation can have the effect of restricting the corrosion resistance and integrity of the weld seam. In order to establish whether the region to be worked has been sufficiently flooded with a shielding gas, it is necessary to monitor the residual oxygen content at the welding site continuously.

In the case of the welding method known from the prior art, oxygen sensors are generally used for detecting the oxygen content of the shielding atmosphere. Thus, the measured oxygen concentration values conventionally serve only for providing the all-clear for starting and are not processed any further. The measurement data are generally also not correlated with the welding progress, and it is consequently difficult or even impossible to obtain a detailed analysis of the welding operation or subsequently to draw conclusions about the welding process.

On account of the aforementioned problem addressed, the present invention is based on the object of providing a device that allows conclusions about the quality of the weld seam to be drawn and a basis for optimization to be created for further welding operations.

With regard to the device, this object is achieved according to the invention as defined in claim 1.

Accordingly, the device according to the invention for measuring and collecting oxygen concentration data is defined by the fact that the device according to the invention has a control unit, which is designed for storing oxygen concentration data during a welding process.

The advantages of the device according to the invention are obvious. For instance, the data collected can be processed in many different ways. New possibilities for drawing conclusions about the welding process are opened up. For example, the data can be used for making new findings with respect to the actually occurring and/or maximum possible oxygen concentration. In particular, the data can be used as a demonstration of quality and for quality assurance. Consequently, not only a high welding quality, but also an optimizable welding process can be achieved.

Advantageous developments of the device according to the invention can be taken from the dependent claims.

It is thus provided in a first embodiment that the at least one sensor element is designed as an optical oxygen sensor. This type of sensor is particularly precise and can without any problem collect oxygen concentration data at high temperatures in the direct vicinity of the welding carried out. The at least one oxygen sensor may of course also be accommodated in any other desired position within the welding chamber and measure the oxygen concentration during the welding process.

Optical oxygen sensors are based for example on the principle of the light adsorption of oxygen atoms preferably at a wavelength of 760 nm. For this purpose, a beam of light of a corresponding wavelength and known intensity is passed through the shielding atmosphere and the loss of intensity is ascertained. On the basis of these data, the oxygen content can then be determined.

An optical method corresponding to the invention as claimed is the fluorescence quenching of a fluorescent medium by oxygen atoms. This involves utilizing the phenomenon known as the "oxygen quenching process". Special organic molecules are excited with light of a suitable wavelength, and so begin to luminesce. This luminescence can be quenched by collisions with other suitable molecules, or oxygen molecules (quenching). This means that the deactivation of the photochemically excited luminophores then takes place in a radiationless manner, i.e. without emitting photons. In the special case of fluorescent media, this is referred to as fluorescence quenching. On the basis of this quenching, the oxygen content of the shielding atmosphere can be determined.

In an alternative example, it is provided that the at least one sensor element is designed as a zirconium dioxide sensor. Zirconium dioxide sensors can represent a low-cost solution for measuring the oxygen content in the welding chamber. They operate on the Nernst principle. Thin layers of platinum are applied on both sides of a zirconium dioxide membrane and serve as electrodes. The zirconium dioxide membrane and the electrodes separate the measurement gas (here shielding gas) from the ambient air. If the zirconium dioxide layer is heated to over 350°C, it becomes an oxygen ion conductor. As long as there is a difference between the oxygen concentration on the two sides of the zirconium dioxide membrane, the oxygen ions migrate from the side of higher oxygen partial pressure to the side of lower oxygen partial pressure, ultimately causing a voltage to drop across the two electrodes. This voltage is a measure of the oxygen partial pressure of the measurement gas (shielding gas).

According to a further example, the at least one zirconium dioxide sensor may be positioned as an additional control sensor along with the optical sensor in the welding chamber. By using a number of sensors, which operate in different ways, the measured values become more reliable. In this way, the system is designed in a redundant manner and is consequently safeguarded against failure.

According to a further aspect of the present invention, the control unit communicates with the at least one sensor element and the welding apparatus via a data line and/or via a wireless LAN. The control unit may in this case serve as a central branch for recording and processing all the measurement data. Furthermore, the control unit may have additional interfaces for additional sensor and/or control elements. Since the control unit contains all the measurement and/or control data of the welding process, these data can be retrieved externally via any desired computer and/or be provided directly to a customer by way of a connection to the Internet (with a data line and/or over a wireless LAN).

According to a further embodiment of the device according to the invention, the control unit has an oxygen concentration database, which is designed for recording the oxygen concentration in dependence on a free variable during the welding operation. However, it is also conceivable to store other measured values, such as for example the temperature, atmospheric humidity, air pressure, etc., in dependence on a free variable. The database used for this purpose may be realized for example as a tabular database in the form of an Excel table. However, other suitable database formats are also possible. The intervals of the measured value entries can be selected individually by the user.

The free variable may for example be the position along the weld seam. This coordinate may have the starting value "X1, Y1" and be recorded in any desired increments together with the associated measured oxygen concentration values by the control unit during the welding operation. This may involve recording the welding progress by way of position sensors and/or calculating the welding progress by way of the speed of the welding apparatus. In the case of orbital welding operations, radial location coordinates in dependence on the welding angle are also possible. Other coordinates determining the location are also conceivable.

According to a further embodiment, the free variable may be the time. The recording of the measured values of the at least one oxygen sensor begins with the starting of the welding operation and may be repeated at any desired time intervals. The recording of the measured values in dependence on the time may comprise not only the local time but also the welding time of the individual welding operation, including a recording of the measured values that is dependent on the time of day and the date. With such a time-dependent recording, long-term conclusions about production as a whole and variations in its quality can be drawn. It is thus possible for example to draw seasonal or weather-related conclusions about the welding process.

With particular preference, the free variable may be a consecutive object number associated with an object to be welded. In the case of small welding jobs and/or a high number of objects to be welded, an object-dependent measurement of the oxygen content of the shielding atmosphere may be relevant and/or adequate.

In this case, only one value is respectively recorded in the database for each object to be welded. This value may be the oxygen concentration value measured at the beginning of the welding operation, a mean value or the maximum variance that has occurred during the welding operation. Of course, a combination of object-dependent, time-dependent and location-dependent measurement and recording is also possible.

According to a further aspect of the present disclosure, the free variable may be freely selected by a user on the control unit from a predefined multiplicity of variables. The control unit could in this case be set and operated digitally, for example by way of a touch control panel, via an external computer, and/or analogly, for example by way of a switch on the welding apparatus and/or on the control unit. In this way, the changing requirements of the objects to be welded can be met quickly and individually.

According to a further embodiment of the device according to the invention, the control unit is designed for controlling the welding operation in dependence on the oxygen concentration. For this purpose, the control unit for example compares prescribed and/or prescribable setpoint values with the measured values and, from the result of the comparison, can influence the welding process directly and/or indirectly. For example, in the case of an oxygen concentration that is too high, further shielding gas can be introduced into the welding chamber. On the other hand, the welding speed can also be adapted individually or, in an extreme case, the welding process can be stopped and/or interrupted. However, it is also conceivable that the control unit controls the welding current and/or, depending on the type of welding, the material feed (for example welding wire) in dependence on the oxygen concentration. The control values can be output by way of signaling devices. It can thus be seen straightaway for example if the welding operation is interrupted on account of an irregularity.

According to a further aspect of the present invention, the control unit is designed for evaluating and/or printing the oxygen concentration data. In this case, the control unit compares the collected measured values with the prescribed values and/or conditions and can evaluate the individual welding processes, but also a long-term development of the welding unit. On the basis of the evaluation of the long-term development, it is possible for example to prevent a possible operating downtime, in which for example relevant wearing parts are exchanged and/or serviced. These evaluations may be output by way of an external and/or an integrated printer.

In a further embodiment of the device according to the invention, the control unit is designed for being programmed by a user, in particular in order to program new variables, closed-loop or open-loop control operations and/or new setpoint values. The programming of new values and/or conditions may take place with a computer connected via a data interface and/or directly by way of the control panel of the control unit. In this case, the programming languages known to a person skilled in the art can be used.

According to a further embodiment of the present invention, the control unit is designed for monitoring and/or controlling the welding process as an autonomous unit outside or inside the welding apparatus. If the control unit is arranged outside the welding apparatus, cableless communication with the sensors and the welding apparatus is appropriate for example. Internally fitted control units, on the other hand, may for example be connected to the sensors and the welding apparatus directly via a data line. The control unit may for example also be designed as an independent, retrofittable unit, and consequently be integrated in already existing welding apparatuses/welding processes, in order to bring them up to the latest state of the art. The control unit may, however, also be a fixed component part of new welding apparatuses.

The invention also relates to a welding unit, in particular an orbital welding unit, that has a device based on the properties described above.

## Claims

1. A device for measuring and collecting oxygen concentration data in welding processes, in particular in shielded arc welding, the device having at least one sensor element for sensing the oxygen concentration of a shielding atmosphere, wherein the device has a control unit, which is designed for storing oxygen concentration data during a welding process,
**characterized in that**
the at least one sensor element has an optical oxygen sensor with a fluorescent media, wherein the functionality of the optical oxygen sensor is based on the oxygen quenching process, wherein
the control unit has an oxygen concentration database, which is designed for recording the oxygen concentration in dependence on a free variable during the welding operation.

2. The device as claimed in claim 1,
the at least one sensor element having a zirconium dioxide sensor.

3. The device as claimed in claim 1 or 2,
the control unit being designed for communicating with the at least one sensor element and the welding apparatus via a data line and/or via a wireless LAN.

4. The device as claimed in claims 1 to 3,
the free variable being the time and/or the coordinates of the weld seam.

5. The device as claimed in claims 1 to 3,
the free variable being a consecutive object number associated with an object to be welded.

6. The device as claimed in claims 1 to 3,
the free variable being freely selectable on the control unit from a predefined multiplicity of variables.

7. The device as claimed in claims 1 to 6,
the control unit being designed for controlling the welding operation in dependence on the oxygen concentration.

8. The device as claimed in claims 1 to 7,
the control unit being designed for evaluating and/or printing the oxygen concentration data.

9. The device as claimed in claims 1 to 9,
the control unit being designed for being programmed by a user, in particular in order to program new variables, closed-loop or open-loop control operations and/or new actual or setpoint values.

10. The device as claimed in claims 1 to 9,
the control unit being designed for monitoring and/or controlling the welding process as an autonomous unit outside or inside the welding apparatus.

11. A welding unit, in particular an orbital welding unit, that has a device as claimed in one of claims 1 to 10.

## Patentansprüche

1. Vorrichtung zum Messen und Sammeln von Sauerstoffkonzentrationsdaten in Schweißvorgängen, insbesondere beim Schutzgasschweißen, wobei die Vorrichtung mindestens ein Sensorelement zum Erfassen der Sauerstoffkonzentration einer Schutzatmosphäre besitzt, wobei die Vorrichtung eine Steuereinheit besitzt, die ausgelegt ist, Sauerstoffkonzentrationsdaten während eines Schweißvorgangs zu speichern,
**dadurch gekennzeichnet, dass**
das mindestens eine Sensorelement einen optischen Sauerstoffsensor mit einem fluoreszierenden Medium besitzt, wobei die Funktionalität des optischen Sauerstoffsensors das Sauerstoffabschreckverfahren als Grundlage verwendet, wobei
die Steuereinheit eine Sauerstoffkonzentrationsdatenbank besitzt, die zum Aufzeichnen der Sauerstoffkonzentration in Abhängigkeit von einer freien Variablen während des Schweißvorgangs ausgelegt ist.

2. Vorrichtung nach Anspruch 1, wobei
das mindestens eine Sensorelement einen Zirkondioxidsensor besitzt.

3. Vorrichtung nach Anspruch 1 oder 2, wobei
die Steuereinheit ausgelegt ist, mit dem mindestens einen Sensorelement und der Schweißvorrichtung mittels einer Datenleitung und/oder mittels eines drahtlosen LAN zu kommunizieren.

4. Vorrichtung nach einem der Ansprüche 1 bis 3, wobei
die freie Variable die Zeit und/oder die Koordinaten der Schweißnaht ist.

5. Vorrichtung nach einem der Ansprüche 1 bis 3, wobei
die freie Variable eine fortlaufende Gegenstandsnummer ist, die einem zu schweißenden Gegenstand zugeordnet ist.

6. Vorrichtung nach einem der Ansprüche 1 bis 3, wobei
die freie Variable in der Steuereinheit aus mehreren vordefinierten Variablen frei wählbar ist.

7. Vorrichtung nach einem der Ansprüche 1 bis 6, wobei
die Steuereinheit ausgelegt ist, den Schweißvorgang in Abhängigkeit von der Sauerstoffkonzentration zu steuern.

8. Vorrichtung nach einem der Ansprüche 1 bis 7, wobei
die Steuereinheit ausgelegt ist, die Sauerstoffkonzentrationsdaten auszuwerten und/oder zu drucken.

9. Vorrichtung nach einem der Ansprüche 1 bis 9, wobei
die Steuereinheit ausgelegt ist, durch einen Anwender programmiert zu werden, insbesondere um neue Variablen, Steuervorgänge mit geschlossenem Regelkreis oder mit offenem Regelkreis und/oder neue Ist- oder SollWerte zu programmieren.

10. Vorrichtung nach einem der Ansprüche 1 bis 9, wobei
die Steuereinheit ausgelegt ist, den Schweißvorgang als eine autonome Einheit außerhalb oder innerhalb der Schweißvorrichtung zu überwachen und/oder zu steuern.

11. Schweißeinheit, insbesondere Orbitalschweißeinheit, die eine Vorrichtung nach einem der Ansprüche 1 bis 10 besitzt.

## Revendications

1. Dispositif destiné à mesurer et à recueillir des données de concentration en oxygène lors de processus de soudage, en particulier en soudage à l'arc sous protection, le dispositif étant doté d'au moins un élément de capteur servant à détecter la concentration en oxygène d'une atmosphère de protection, le dispositif étant doté d'une unité de commande, qui est conçue pour stocker des données de concentration en oxygène pendant un processus de soudage,
**caractérisé en ce que**
l'élément ou les éléments de capteur sont dotés d'un capteur optique d'oxygène comportant un milieu fluorescent, la fonctionnalité du capteur optique d'oxygène étant basée sur le processus d'extinction de l'oxygène,
l'unité de commande étant dotée d'une base de données de concentration en oxygène, qui est conçue pour enregistrer la concentration en oxygène en fonction d'une variable libre pendant l'opération de soudage.

2. Dispositif selon la revendication 1,
l'élément ou les éléments de capteur comprenant un capteur au dioxyde de zirconium.

3. Dispositif selon la revendication 1 ou 2,
l'unité de commande étant conçue pour communiquer avec l'élément ou les éléments de capteur et l'appareil de soudage via une ligne de données et/ou via un LAN sans fil.

4. Dispositif selon les revendications 1 à 3,
la variable libre étant le temps et/ou les coordonnées du cordon de soudure.

5. Dispositif selon les revendications 1 à 3,
la variable libre étant un numéro d'objet consécutif associé à un objet à souder.

6. Dispositif selon les revendications 1 à 3,
la variable libre étant librement sélectionnable sur l'unité de commande parmi une multiplicité prédéfinie de variables.

7. Dispositif selon les revendications 1 à 6,
l'unité de commande étant conçue pour commander l'opération de soudage en fonction de la concentration en oxygène.

8. Dispositif selon les revendications 1 à 7,
l'unité de commande étant conçue pour évaluer et/ou imprimer les données de concentration en oxygène.

9. Dispositif selon les revendications 1 à 9,
l'unité de commande étant conçue pour être programmée par un utilisateur, en particulier afin de programmer de nouvelles variables, des opérations de régulation en boucle fermée ou en boucle ouverte et/ou de nouvelles valeurs réelles ou de consigne.

10. Dispositif selon les revendications 1 à 9,
l'unité de commande étant conçue pour surveiller et/ou réguler le processus de soudage en tant qu'unité autonome à l'extérieur ou à l'intérieur de l'appareil de soudage.

11. Unité de soudage, en particulier unité de soudage orbital, dotée d'un dispositif selon l'une des revendications 1 à 10.
